# EUROPEAN PATENT APPLICATION

(11) **EP 2 907 539 A1**
(43) Date of publication of application: **19.08.2015**
(21) Application number: 14275245.0
(22) Date of filing: 24.11.2014
(51) Int. Cl.: A61M 25/06, A61M 25/01

(54) **Sheath or catheter for medical introducer assembly**

(30) Priority: 12.02.2014 GB 201402470
(71) Applicant: Cook Medical Technologies LLC, Bloomington, IN 47404 (US)
(72) Inventor: Christiansen, Frank, 4690 Allegade (DK)
(74) Representative: Williams Powell

(57) **Abstract**

An introducer assembly (10) provided with a sheath (16) formed of a plurality of elongate sections (22-26) which are coupled to one another along their longitudinal sides (28,30) by a dovetail coupling (32,34). The sheath sections (22-26) are slidable longitudinally relative to one another, enabling the sheath (16) to be retracted in stages. This reduces the amount of friction which must be overcome in the use of the sheath (16) and in particular during the deployment of a medical device carried within the sheath (16). The structure can also substantially increase the precision of deployment of the medical device, as well as decreasing the force required during the deployment operation.

## Description

### Technical Field

The present invention relates to a sheath or catheter for a medical introducer assembly, and to an introducer assembly including such a sheath or catheter.

### Background Art

Medical introducer assemblies are in common use for the deployment of medical equipment or devices in a patient, and in particular for vascular implantation of medical devices such as stents, stent grafts, vena cava filters, occlusion devices, prosthetic valves and so on. Generally, such assemblies are fed endoluminally through the patient's vasculature to the treatment site, often over significant distances. For instance, aortic devices may be introduced via the femoral artery and fed all the way up to the aortic arch and even the ascending aorta to the heart. Such introducer assemblies can have lengths of well over one metre, in some cases up to two metres or more. Even assemblies intended to be fed into the patient at a distance closer to the treatment site will generally have significant lengths.

Introducer assemblies of this nature include an outer sheath which houses the majority of the elements of the assembly, as well as the medical device, treatment or diagnostic tool. The distal end of the sheath normally has a dilator tip extending therefrom, for assisting in guiding the sheath through the patient's vasculature. The proximal end of the sheath typically remains outside the patient and is connected to a haemostatic valve assembly and deployment triggers. The sheath is generally a tight fit to the components it houses in order to ensure that these are securely held during the deployment process, as well as to ensure that the sheath has the smallest practical outer diameter. The sheath needs to be sufficiently flexible yet sufficiently strong to have good trackability through often tortuous vasculature, as well as good pushability to get its distal end reliably at the treatment site. In most cases the introducer assembly and thus the sheath will be caused to curve, often several times along its length, as a result of curvatures and bifurcations in the vasculature. These characteristics of the sheath and its use create friction within the sheath between the inner wall of the sheath and the components it houses, most typically with the medical device or tool or the elements of the assembly which hold the latter in place at the distal end of the sheath. This friction must be overcome in order to pull back the sheath so as to expose the medical device or tool, which often requires a significant force by the clinician. The application of such a force, however, makes it difficult to hold the distal end of the introducer assembly steady, particularly given that the only part of the assembly which can be held is its proximal end which remains outside the patient. As a result, the distal end can often jump out of position, leading to the risk of misplacement of the medical device or tool. This problem is often exacerbated with medical devices which self-expand and which will thus be pressing against the inner wall of the sheath when held they are held inside it.

Further difficulties can arise with medical devices and tools which are orientation dependant, such as fenestrated or branched medical devices, and which must not only be disposed in the correct longitudinal position in a vessel but also in the correct orientation.

Attempts have been made in the art to alleviate such drawbacks, for instance with splittable or expandable sheaths, but such solutions are not always appropriate and do not necessarily alleviate the problems mentioned above.

Another issue with such assemblies lies with the provisions necessary to accommodate the guide wire which is often used for placing the introducer assembly in the patient. This is normally fed into the patient first, with the introducer assembly then fed over the guide wire, which acts to train the distal end of the assembly around the patient's vasculature between the percutaneous entry point and the treatment site. The guide wire will generally be kept in place in the patient for the duration of the medical procedure, particularly important when the procedure involves multiple steps and thus the use of multiple introducer assemblies or components. In order to be able to hold the guide wire in position, this will often have to be of significant length, typically greater than two times the length of the sheath. This can often lead to the guide wire having very significant lengths.

Known designs of sheath can be found, for example, in US-8,088,154 and US-2010/0,049,305.

### Disclosure of the Invention

The present invention seeks to provide an improved sheath or catheter for an introducer assembly and to an improved introducer assembly.

According to an aspect of the present invention, there is provided a sheath or catheter for a medical introducer assembly including a plurality of elongate sections, each having first and second longitudinal sides and couplings at said first and second longitudinal sides; said elongate sections being connected together by said couplings to form the sheath or catheter; said couplings allowing longitudinal movement of elongate sections coupled thereby, such that the elongate sections are movable relative to one another.

Such a structure enables the sheath to be retracted in stages, such that only a portion of the circumferential extent of the sheath is moved at a time, and thus only a portion of the internal surface of the sheath slides along the internal components of the sheath. The reduced surface area of sheath surface which moves at any one time will present significantly reduced friction to movement and will thus require a significantly reduced deployment force. Moreover, the ability to move elongate sections independently of one another enables the clinician to hold steady the position of the remaining sheath sections, and thus the distal end of the introducer assembly, while retracting one section. The assembly can thus be supported so as to improve positioning accuracy.

Advantageously, the elongate sections are transversally curved, for instance part-rounded. Thus, the sheath or catheter may be round in transverse cross-section, as with a conventional sheath, although it may be non-round, for instance oval. It is to be appreciated that the teachings herein are not limited to a sheath of any specific cross-sectional shape.

The sheath or catheter may be formed of two elongate sections, although in the preferred embodiment has three sections. Other embodiments may have four or more elongate sections.

The elongate sections may have equivalent transverse dimensions, so as to be of the same circumferential size, but in other embodiments they may have different transverse sizes. For instance, there may be provided one or more elongate section which is/are narrower than the others.

The couplings preferably extend for a least a part of said length and most preferably extend for the whole of the length. The couplings advantageously lock the elongate sections together in the transverse direction, thus to prevent radial splitting or separation of the elongate sections from one another.

Preferably, the couplings include a first coupling type providing a channel with a narrowed opening and a second coupling type providing a rib or series of protrusions shaped to fit into the channel. The couplings are most preferably dovetail couplings.

The elongate sections preferably include proximal and distal ends and are provided with gripping elements at their proximal ends. These may be tabs or the like or form part of a mechanism such as a handle for gripping and then manipulating the elongate elements.

Advantageously, the elongate sections are provided with strengthening elements.

The sheath or catheter may be rigid, so as not to be curved during use, as it may be flexible so as to be able to pass through a patient's vasculature in an endoluminal deployment procedure.

According to another aspect of the present invention, there is provided an introducer assembly for the deployment of medical devices or tools, the assembly including a sheath or catheter, the sheath or catheter including a plurality of elongate sections each having first and second longitudinal sides and couplings at said first and second longitudinal sides, said elongate sections being connected together by said couplings to form the sheath or catheter, said couplings allowing longitudinal movement of elongate sections coupled thereby, such that the elongate sections are movable relative to one another.

It is not necessary with such a catheter or sheath to provide a pusher rod for holding the medical device in position while retracting the cheat or catheter.

According to another aspect of the present invention, there is provided a method of deploying an implantable medical device or a treatment or diagnostic tool by means of an introducer assembly which includes a sheath or catheter, the sheath or catheter having a proximal end and a distal end and including a plurality of elongate sections each having first and second longitudinal sides and couplings at said first and second longitudinal sides, said elongate sections being connected together by said couplings to form the sheath or catheter, said couplings allowing longitudinal movement of elongate sections coupled thereby, such that the elongate sections are movable relative to one another; the method including the steps of:
disposing the distal end of the sheath or catheter in a patient;
retracting one of said elongate sections while holding the other elongate section or sections in position, so as to expose a part of the medical device or tool; and
retracting the other elongate section or sections while holding said one elongate section in position, thereby to expose the remainder of the medical device or tool.

It will be appreciated that the sheath or catheter disclosed herein is particularly suitable as the outermost sheath of an introducer assembly.

Other features and advantages of the teachings herein will become apparent from a consideration of the specific description which follows.

### Brief Description of the Drawings

Embodiments of the present invention are described below, by way of example only, with reference to the accompanying drawings, in which:
Figure 1 is a schematic view of an embodiment of introducer assembly;
Figure 2 is a perspective view of the distal end of a preferred embodiment of sheath;
Figure 3 is a transverse cross-sectional view of one section of the sheath of Figure 2;
Figure 4 is a perspective view of a part of one section of the sheath of Figure 2;
Figure 5 is a transverse cross-sectional view of another embodiment of coupling arrangement for the sheath sections;
Figure 6 is a perspective exploded view of another embodiment of coupling arrangement for the sheath sections;
Figure 7 is a transverse cross-sectional view of another embodiment of coupling arrangement for the sheath sections;
Figure 8 is a transverse cross-sectional and perspective view of the components of another embodiment of coupling arrangement for the sheath sections;
Figure 9 is a schematic view of an embodiment of the proximal end of the sheath of Figure 2;
Figures 10 to 11B are schematic views of various other embodiments of the proximal end of the sheath; and
Figure 12 is a schematic view of the sheath of Figure 2 shown partially retracted.

### Description of the Preferred Embodiments

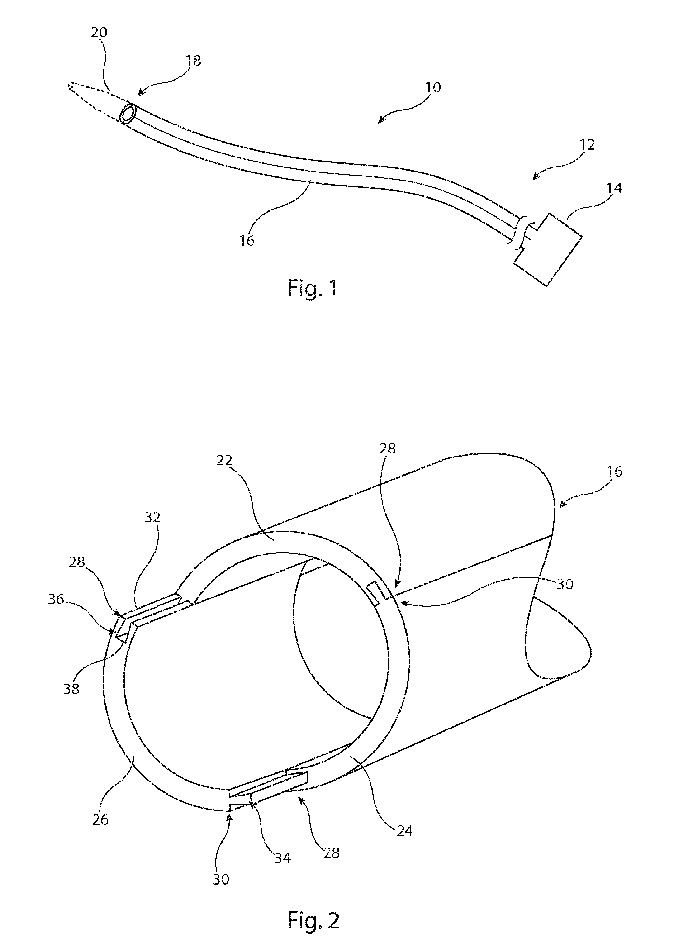

Referring to Figure 1, this shows in schematic form an embodiment of introducer assembly 10 according to the teachings herein. The introducer assembly may be used for implanting a medical device into a patient, for example a stent, stent graft, vena cava filter, occluder, embolization device, prosthetic valve and so on. It may also be used for deploying diagnostic or other medical treatment equipment. Introducer assemblies suitable for such medical purposes are generally known in the art.

The assembly 10 includes a proximal end 12 provided with a manipulation unit 14, which typically includes one or more haemostatic valves, couplings for the administration of flushing fluid, control elements for controlling the operation of the assembly 10, such as for effecting the deployment of the device carried in the assembly, and so on. Such elements are known in the art and are therefore not described in further detail herein.

The assembly also includes a sheath 16, described in detail below, which extends from the manipulation unit 14 to a distal end 18 of the assembly 10. The sheath may have a length from several tens of centimetres to well over one metre, even up to two metres or more, in dependence upon the intended location of percutaneous insertion of the distal end of the assembly 10 into the patient, which is often far from the treatment site. The majority of the sheath 16 will be fed into the patient during the procedure and it thus needs to be flexible in order to be able to curve around the patient's vasculature (that is have good trackability), yet sufficiently strong to be able to transmit force applied thereto from the manipulation unit 14 to its distal end 18 so as to push through the vasculature up to the treatment site (that is to have good pushability).

The distal end 18 of the assembly 10 typically has a dilator tip 20, although this is not always necessary.

The sheath 16 provides an internal lumen 15 for housing the components of the assembly and in particular the device to be deployed (not shown) and the elements required to effect such deployment. The device is typically held at or close to the distal end 18 of the assembly 10 and the deployment elements, which may include a device carrier and a device pusher element, are generally located at or proximal of the medical device. It is important that the sheath 16 is a close fit, often a tight fit, over the components it houses for a number of reasons. First, the sheath 16 should have as small an outer diameter as possible, which optimises its trackability. Secondly, the sheath needs to hold the components it houses securely therewithin, leaving little or no room for these components which might enable them to become dislodged during the medical procedure and other handling of the assembly. This is particularly important in the case of orientationally dependent medical devices, for example, branched or fenestrated medical devices. Third, the sheath often plays a part in radially constraining the medical device, in particular devices which are self-expanding.

This close or tight fit, added to the fact that the sheath is not normally straight at the moment of deployment of the medical device, contributes to significant friction between the internal wall of the sheath and the components it houses. A large force is required to overcome this friction, particularly at the start of the deployment procedure, which can lead to a number of complications, including jolting of the distal end 18 of the assembly 10 and thus loss of the positioning of the medical device, deformation of the medical device, and so on.

The sheath 16 of this embodiment of assembly 10 has a segmented structure, described in detail below, which enables the sheath 16 to be withdrawn in sections rather than as a whole as with prior art sheaths.

A better view of the preferred embodiment of sheath 16 can be seen in Figure 2, which shows the distal end 18 of the sheath 16, in a condition in which two sections thereof have been partially retracted.

The sheath 16, in this embodiment, has a conventional round shape in axial cross-section, although in other embodiments the sheath 16 could have other transverse cross-sectional shapes, such as oval. The shape of the sheath 16 in transverse cross-section is not material to the teachings herein.

The sheath 16 of this embodiment is formed of three elongate sections 22, 24, 26 which each, in this example, rounded in transverse cross-section, and in particular being third-circular. Each section 22-26 includes a first longitudinal side 28 and a second longitudinal side 30. In the embodiment shown in Figure 1, the sections 22-26 are the same as one another. Each longitudinal side 28 is provided, in this embodiment, with a channel 32 extending along the length of its respective section 22-26, while the each longitudinal side 30 is provided with an elongate rib 34, equally extending along the length of its respective elongate section 22-26. The rib 34 is shaped and sized to fit within the elongate channel 32 of the adjacent section 22-26 and in the preferred embodiment in such a way that the rib 34 interlocks with the walls 36, 38 of the side 28 which provide the channel 32, as explained in further detail below. The structure is such that the ribs 34 are able to slide in their respective channels 32, and thereby such that the sections 22-26 are able to slide relative to one another. Figure 2 shows the sections 22 and 24 partially retracted relative to the section 26.

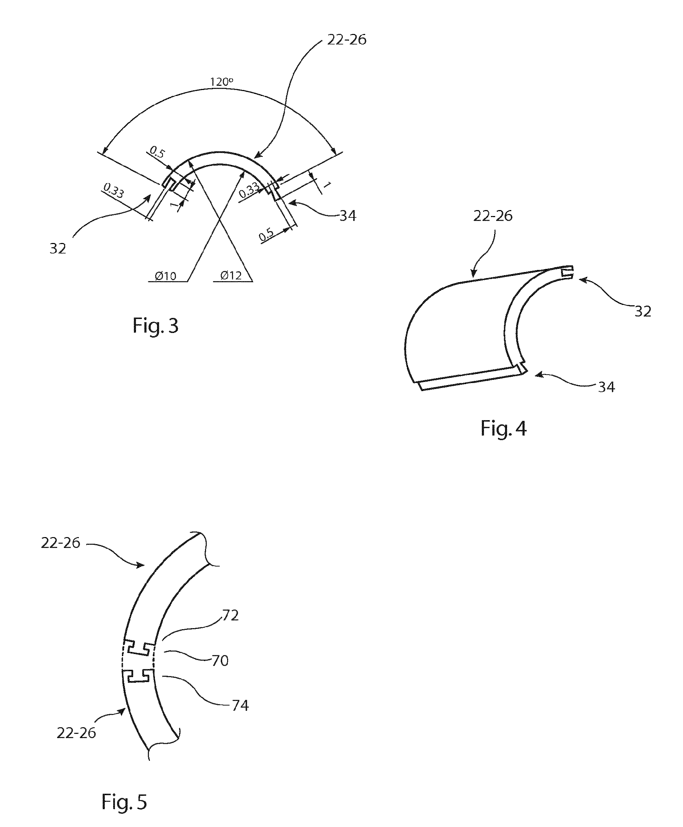

Referring now to Figures 3 and 4, these show in better detail the structure of the elongate sections 22-26 of the sheath 10, with preferred dimensions for a sheath having an inner diameter of 10mm and an outer diameter of 12mm. As will be apparent, the ribs 34 have a dovetail shape, with the channels 32 being similarly shaped to receive the dovetail ribs 34. This shape, as will be appreciated can ensure that the ribs 34 cannot be pulled laterally out of the channels 32, thereby ensuring that the sheath 16 remains intact during its use. In this example, the tape of the dovetail ribs 34 and equivalent dovetail recess 32 is of around 33% reduction. It has been found that this provides good radial strength preventing radial separation of the sections 22-24, yet good sliding qualities. Other tapers are envisaged, particularly of greater than 33% reduction.

It will be appreciated that the shape of the ribs 34 and the corresponding shape of the recesses 32 can be other than dovetailed as shown. In particular, they could be any shape which enables the sections 22-26 to slide longitudinally relative to one another and yet which prevents uncoupling of the sections 22-26 from one another in the circumferential/radial direction (such as by being pulled apart). In another embodiment, the ribs 34 may have an enlarged head or beading with a narrower neck fixed to the side 30 and correspondingly shaped channels. In another example, the ribs 34 could have a laterally extending flange, and the channels 32 provided with corresponding laterally extending side channels, again to provide the circumferential/radial locking function.

The sheath 10 and in particular the sections 22-26 are made of a flexible material, so as to give the sheath 16 good trackability and yet of strength sufficient to give the sheath the necessary pushability. Preferred materials include polyamide, polyether block amide such as Pebax™, polyethylene terephthalate (PET), high-density polyethylene (PEHD), polyurethane, polyimide (PI) or polytetrafluoroethylene (PTFE).

The sections 22-26 of the sheath 16 are preferably made of a single layer of material but in other embodiments may have incorporated therewithin strengthening elements, such as braiding, curved strips of metal and so on. These would typically be embedded within the wall of the sections 22-26.

The connection elements 32, 34 may be made of a polymer extruded to a PTFE sublayer, in practice of any suitable material which provides radial strength to the couplings and thus to the assembly could be used. Various examples are given below.

The sheath 16 may be coated on its internal and/or its external surfaces with PTFE (Teflon). The coupling elements may similarly be coated.

Figures 5 to 8 show different embodiments of connection element structures for the sheath 16 taught herein. Referring first to Figure 5, the sections 22-26 are provided with extruded connector profiles 70, 72, with the profile 70 providing a substantially flat strip coupled to the section wall by a narrower neck 72, whereas the female connection element 74 provides a correspondingly-shaped channel therein.

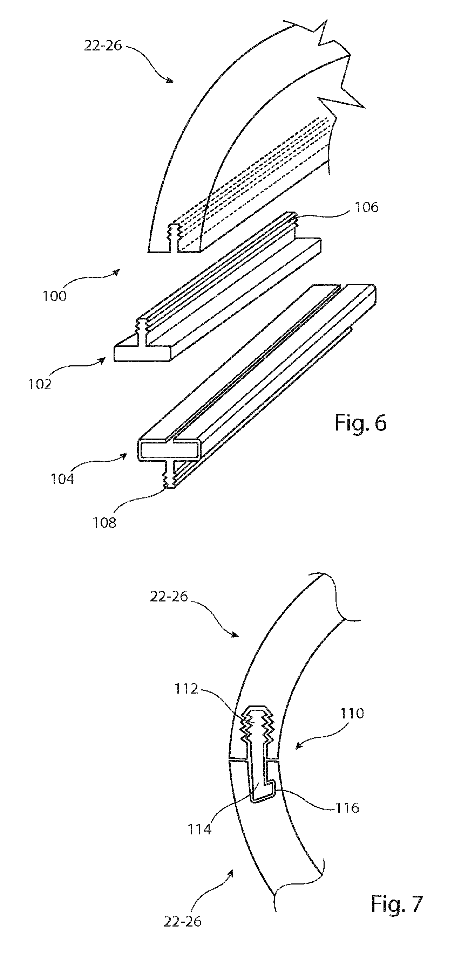

The example of Figure 6 has connection elements of similar shape to the example of Figure 5 but which is provided with strengthened inserts 102 and 104, made for example of metal or other substantially rigid material, and which have ribbed flanges 106, 108 which engage into corresponding channels in the sheath sections 22-26 and are able to embed into the material of those channels. The inserts 102 and 104 may be made of any material harder than the material of the walls of the sheath sections 22-26. It will be appreciated that in circumstances where the inserts 102, 104 are made of a rigid material, the sheath 16 will be substantially straight and can thus be used, for example, in open surgery procedures where it is not necessary to cause the sheath 16 to curve around the patient's vasculature or organs. It is not excluded, though, that the inserts 102, 104 could be made of a flexible material.

With reference to Figure 7, this embodiment provides a single insert 110 having a ribbed flange 112 the equivalent to the flanges 106 and 108 of the embodiment of Figure 6 and a hooked flap 114 which extends at an angle of approximately 90° to the rib. The fixed flap 114 is able to slide within a correspondingly-shaped channel 116 in the edge of the adjacent sheath section 22-26.

Another advantage of the inserts is that it is difficult to manufacture parts by extrusion with very tight tolerances. Inserts can avoid this problem given that they can be accurately manufactured or machined.

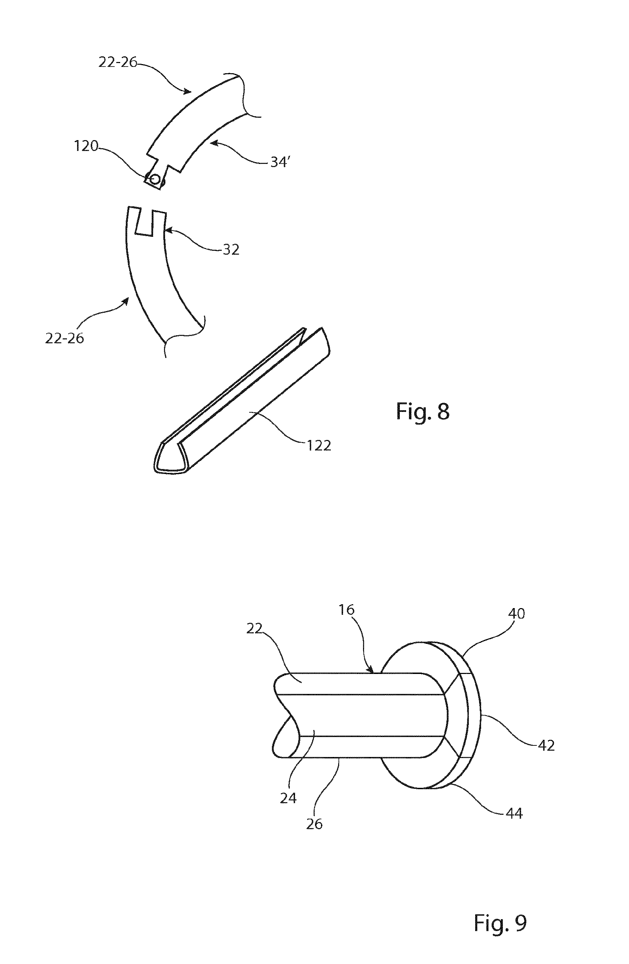

With reference to Figure 8, this embodiment provides within the channel 32 of each sheath section 22-26 an insert 122, which may be made of metal or other rigid other substantially rigid material, which is shaped to have a narrower opening at its base. The rib 34' of each sheath section 22-26 includes a reinforcement element such as a wire extending through the rib close to its extremity. The reinforcement wire preferably has a diameter similar to or larger than the opening of the insert 122 so as to ensure that the coupling elements of the adjacent sheath sections 22-26 remain firmly connected together during use. The reinforcement wire 120 can also cause the outer walls of the rib 34' to bulge slightly, which can have the effect of reducing the surface contact between the rib 34 and the insert 122, thereby to reduce friction. It will be appreciated that the reinforcement wire 120 and/or insert 122 can readily be made of a material which is simply harder than the material forming the walls of the sheath sections 22-26.

Referring now to Figure 9, there is shown an example of structure for the proximal end of the sheath 16. The elongate sections 22-26 are coupled, in this example, to respective flanges 40-44 which extend radially outwardly from their respective sections 22. These flanges are fixed to their respective sections and act as gripping elements or handles used in sliding the sections 22-26 relative to one another during the deployment operation. The actual structure of the elements 40-44 is not material, it being simply important to be able to apply force to the sheath sections 22-26 in order to move these relative to one another or, as explained below, to hold one or more of the sections 22-26 in position. The elements 40-44 could be directly used by a clinician or could be incorporated within the manipulation assembly 14 shown in Figure 1 and actuated via appropriate actuators of the manipulation unit 14, in the manner akin to the actuators used in such introducer assemblies for, for instance, operating trigger wires and other medical device restraining elements.

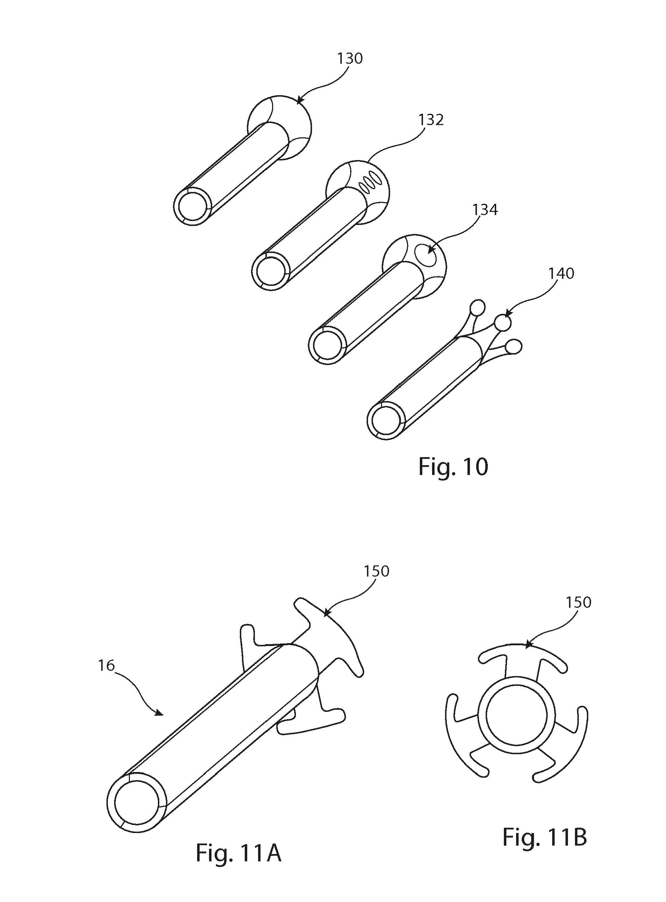

Referring now to Figure 10, there are shown various different examples of gripping elements provided at the proximal end of the sheath 16 for use in gripping and moving the sheath sections 22-26. One example includes a plain grip 130, as in the embodiment of Figure 9, although the flanges of the gripping element 130 may be curved as shown, for user comfort. The example of gripping element 132 provides a plurality of ribs on each flange for reducing any possible risk of sliding of a user's finger during the manipulation of the gripping elements. The example of the gripping element 134 provides a hole or aperture in each flange of a size which can be gripped by the tip of a user's finger. The embodiment of grip element 140 provides a radially outwardly extending tapering tab terminating in a ball or other enlarged head able to be readily gripped by a user.

Figures 11A and 11B show another embodiment of the proximal end of the sheath 16, in which each gripper element 150 is in the shape of a radially outwardly extending T-bar. This is preferably of a size large enough that each T-bar can be gripped between a user's fingers.

The skilled person will appreciate that other designs or structures of gripping element can be used in addition with or instead of the examples shown herein.

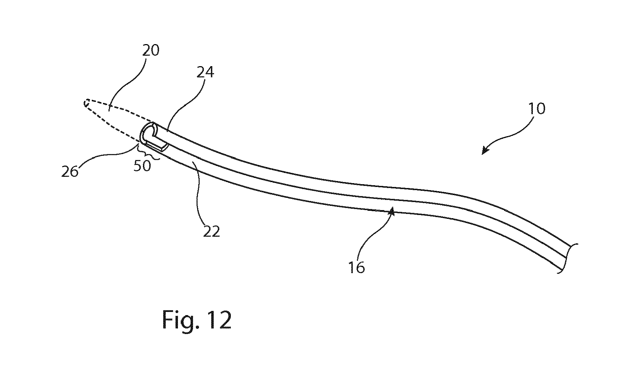

Referring now to Figure 12, there is shown in schematic form the major portion of the introducer assembly 10 of Figure 1, showing in particular the elongate section 22 retracted relative to the sections 24 and 26, achieved, as will be appreciated, by pulling on the flange or other device 40. This retraction of the sheath section 22 partially exposes the elements held within the sheath 16, particularly at its distal end and at the device holding zone 50. In the case where an implantable medical device is located at the distal end of the assembly 10, and thus at the distal end of the sheath 16, the retraction of one of the sheath elements 22 will expose a part of that medical device.

Considering first the issue of friction between the internal wall of the sheath 16 and the devices housed within the sheath 16, only a fraction of that friction needs to be overcome in retracting, in this example, the section 22 and specifically only a third of that friction given that only a third of the internal area of the sheath is moved. Thus, much less force is required to move the section 22 of the sheath 16. Similarly, once one of the sections 22 has been partially retracted, the retraction of any of the other sections 24, 26 will equally require less force as it will be necessary to overcome only a part of the total friction. In practice, once a first section 22 has been retracted, even partially, there is generally likely to be a reduction in the remaining level of friction between the internal walls of the sheath 16 and the components housed therewithin. This would be in part as a result of the fact that the remaining portions 24, 26 of the sheath 16 would be able to expand radially outwardly to a certain extent. In some instances, the components held within the sheath 16 may also be able partly to expand at the zone 50 once the first section 22 has been retracted, leading to further reduction in friction between the sheath 16 and those components.

It is to be appreciated also that in addition to a reduction in the friction which has to be overcome in order to commence the deployment operation, additional support to the sheath 16 is provided by being able to hold the other sheath sections, in this example the sections 24 and26, in position by applying a holding pressure to the proximal ends of the sheath portions 24, 26. In other words, the sheath portions 24, 26 can be held and supported in position while a pulling force is applied to the flange 40 to retract the sheath section 22. This is in addition to being able to apply a similar holding force to the internal components within the assembly 10 and in particular a carrier catheter, pusher element or the like. Being able to hold the sheath 16 in position provides significant advantages in terms of retaining the distal end 18 of the introducer assembly 10 at the correct location and orientation during the commencement of the deployment operation.

Once one of the sheath sections 22 has been partially retracted, the other sheath sections 24-26 can also be retracted, in which case a holding force is applied to the flange 40 of the section 22 and the other sheath section, for example 26, while pulling back on the flange 42 (or other device) to retract the sheath section 24. Thus, each of the sheath sections 22-26 can be retracted in turn so as to expose gradually the entirety of the medical device held at the distal end 18 of the introducer assembly 10. This does, of course, also provide for staged deployment of the medical device held at distal end 18 of the introducer assembly 10 and thus a more gradual and precise deployment and positioning of this within the patient's vasculature.

It will be appreciated also that such sequential retraction of sheath sections 22-26 can be particularly advantageous in the deployment of orientation-specific medical devices or tools, such as branched or fenestrated stent grafts and so on, where, for example, a branch could be exposed by the retraction of a first sheath section 22, positioned as required, before retraction of the remaining sheath sections to deploy the main body of the stent graft or other device. This can provide significant advantages in terms of ease and accuracy of deployment of orientation-specific medical devices and tools.

In addition to the above-described advantages, the provision of a sheath 16 made of elongate sections which are able to slide relative to one another provides better flexibility of the sheath 16 and as a result better trackability within a patient's vasculature, in that the flexibility of the sheath may not be dependent solely upon the flexibility of the material and structure of the walls of the sheath, but is also improved by the fact that the sheath sections 22-26 are able to slide relative to one another as the sheath 16 curves, in particular, to reduce the resistance to curvature of the material. This can also assist in reducing the risk of kinking of the sheath when it is curved to tight angles.

It is not necessary with introducer assembly 10 taught herein to have a pusher rod of the type conventional in the art, since those sections of sheath 16 which are held in place while one of the sections is retracted, will act also to hold the implantable medical device in position as the result of the higher friction between those stationary sheath sections 22-26 and the medical device, relative to the friction between the one sheath section 22-26 which is moved and the medical device. This leads to a simpler introducer assembly and also a simpler deployment procedure. It is to be understood, though, that a pusher element of conventional type could be used with this introducer assembly 10.

The sheath sections 22-26 could be provided with different markings and/or colours, useful in denoting the orientation of the medical device or tool held within the sheath 16, and also for indicating the order of retraction of the individual sheath sections 22-26, for example to expose first a fenestration or side branch to a medical device held within the sheath. In this regard, the partial retraction of the sheath 16 can assist in the correct orientation of an orientation dependent medical device, such as a fenestrated or branched stent graft, in that a first sheath section 22-26 can be pulled back (typically the section overlying the fenestration or side branch), allowing fine positioning of the medical device and in particular the fenestration or side branch given that the major portion of the medical device will be still held in a radially contracted configuration, with that major portion being released from the sheath only once the fenestration or side branch has been correctly positioned in the patient. In this regard, it would also be possible to pass the guidewire through the fenestration or side branch once it has been partially deployed, with the major portion of the medical device still held constrained within the sheath sections which have not yet been retracted.

A further advantage of this structure of sheath lies in the handling of a guide wire used during the deployment operation, as is often used in the art. As explained above, where such guide wire is used and in circumstances where the medical procedure may involve a plurality of phases and, it is often necessary to have a very long guide wire in order to be able to keep this in place during the withdrawal of the sheath 16 from the patient. However, with a sheath 16 having sections 22-26 which can be independently removed, the sheath 16 could be removed from a guide wire by removal of one of the sheath sections 22 and then effectively "peeling off" the remainder the sheath from the guide wire, which can thus be substantially shorter than with conventional single-piece sheaths. An analogy could be made with a rapid exchange introducer arrangement.

It is to be understood that the number of elongate sections 22-26 of the sheath 16 may not be just three as shown. Other embodiments may have a greater or even a lesser number of sheath sections. For example, a sheath could be made of just two sections, whereas it could be made of four sections or even more. Moreover, even though the embodiment described above has sections which are each of the same width, in the example shown third-circular, they need not be the same width. For instance, there may be a narrower section with one or more wider sections, or one wider section with one or more narrower sections. The relative widths of the elongate sections 22-26 will be dependent primarily on the desired characteristics of the sheath 16 and of the device housed within the sheath 16 and to be deployed thereby. For instance, one of the sheath sections 22 may be sized in accordance with the size of a side branch or fenestration.

It will be appreciated that the sheath or catheter disclosed herein is particularly suitable as the outermost sheath of an introducer assembly.

The sheath or catheter taught herein may be made flexible, in which case its various components are made from flexible material. As mentioned above, the sheath or catheter may also be substantially rigid, in which case one or more of its components may be made of substantially rigid material.

In all of the above described embodiments, there may be provided between adjacent sheath sections 22-26 a breakable connection element, such as a tab, or small protrusion and corresponding recess in abutting wall portions of adjacent sheath sections, for holding the sheath sections 22-26 in position relative to one another until a deliberate pulling force is applied to the sheath sections 22-26, thereby to prevent premature sliding movement between the sheath sections.

It is to be understood that the above-described embodiments are examples only of the invention taught herein and that the invention could be embodied in different forms. For example, the teachings herein could also be used in other catheters and cannulas used for medical applications, including rigid cannulas.

It is to be understood that all optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A sheath or catheter for a medical introducer assembly including a plurality of elongate sections each having first and second longitudinal sides and couplings at said first and second longitudinal sides, said elongate sections being connected together by said couplings to form the sheath or catheter, said couplings allowing longitudinal movement of elongate sections coupled thereby, such that the elongate sections are movable relative to one another.

2. A sheath or catheter according to claim 1, wherein the elongate sections are transversally curved.

3. A sheath or catheter according to claim 2, wherein the elongate sections are transversally part-rounded.

4. A sheath or catheter according to claim 1, 2 or 3, wherein the sheath or catheter is round or oval in transverse cross-section.

5. A sheath or catheter according to any one of claims 1 to 4, including two, three, four or more elongate sections.

6. A sheath or catheter according to any one of claims 1 to 5, wherein the elongate sections have equivalent or different transverse dimensions.

7. A sheath or catheter according to any preceding claim, wherein the elongate sections have a length and the couplings extend for a least a part of said length.

8. A sheath or catheter according to any preceding claim, wherein the elongate sections have a length and the couplings extend for the whole of said length.

9. A sheath or catheter according to any preceding claim, wherein the couplings lock said sections together in a transverse direction.

10. A sheath or catheter according to any preceding claim, wherein the couplings include a first coupling type providing a channel with a narrowed opening and a second coupling type providing a rib or series of protrusions shaped to fit into the channel.

11. A sheath or catheter according to any preceding claim, wherein the couplings are dovetail couplings, T-shaped couplings or J-shaped couplings.

12. A sheath or catheter according to any preceding claim, wherein at least one of the couplings includes a reinforcement element.

13. A sheath or catheter according to any preceding claim, wherein the elongate sections include proximal and distal ends and are provided with gripping elements at their proximal ends.

14. A sheath or catheter according to any preceding claim, wherein the elongate sections are provided with strengthening elements.

15. An introducer assembly for the deployment of medical devices or tools, the assembly including as an outermost sheath a sheath or catheter according to any preceding claim.
